# FASCICULE DE BREVET EUROPEEN

(11) **EP 3 280 394 B1**
(45) Date de publication et mention de la délivrance du brevet: **18.11.2020**
(21) Numéro de dépôt: 16722282.7
(22) Date de dépôt: 07.04.2016
(51) Int. Cl.: A61K 9/00, A61K 9/06, A61K 31/095, A61K 31/717, A61K 33/00, A61K 33/14, A61P 17/00, A61P 17/10, A61P 17/06, A61P 33/00, A61P 31/10

(54) **GEL A BASE DE SEL ET SON UTILISATION DANS LA PRÉVENTION ET LE TRAITEMENT DES PARASITOSES ET DES AFFECTIONS DERMATOLOGIQUES**
SALZGEL UND VERWENDUNG DAVON IN PRÄVENTION UND BEHANDLUNG VON PARASITOSEN UND HAUTERKRANKUNGEN
SALT GEL AND USE THEREOF IN PREVENTING AND TREATING PARASITOSES AND SKIN DISORDERS

(30) Priorité: 09.04.2015 FR 1553056
(43) Date de publication de la demande: 14.02.2018
(73) Titulaire: Concept, 75013 Paris (FR)
(72) Inventeur: GRISLAIN, Luc, 33200 Bordeaux (FR); PAGNOTTA, Nadine, 33200 Bordeaux (FR)
(74) Mandataire: Aquinov
(86) Numéro de dépôt international: PCT/FR2016/050798
(87) Numéro de publication internationale: WO 2016/162640

(56) Documents cités:
- EP-A1- 2 756 839
- CA-A1- 2 120 967
- FR-A1- 2 996 453
- US-B1- 6 607 716
- "Licefreee Spray Label Information", Licefreee.com , 2018, Retrieved from the Internet: URL:https://www.licefreee.com/en/resources /product-labels/licefreee-spray-label-info rmation/ [retrieved on 2019-01-16]
- "Licefreee Gel Label Information", Licefreee.com , 2018, Retrieved from the Internet: URL:https://www.licefreee.com/en/resources /product-labels/licefreee-gel-label-inform ation/ [retrieved on 2019-01-16]

## Description

La présente invention se rapporte à une composition sous forme de gel contenant du sel et son utilisation en application topique, sur la peau, les phanères, les muqueuses ou le cuir chevelu, dans la prévention et le traitement des parasitoses et des affections dermatologiques en général.

On sait que certaines pathologies cutanées ou parasitoses peuvent être traitées à l'aide de fortes concentrations de sel.

Ces formulations doivent être hypertoniques c'est-à-dire contenir des concentrations en sel supérieures à 9 grammes/litre. Dans ces conditions, l'eau intracellulaire des organismes vivants (microorganismes ou insectes) va migrer vers le milieu extérieur et provoquer une déshydratation fatale .

Cette propriété peut être mise à profit pour l'éradication des parasites du types poux et *Demodex* notamment. En effet, ces solutions salines concentrées peuvent s'avérer efficaces pour lutter contre des parasitoses dermatologiques causées par des insectes comme le *Demodex* ou les poux.

Le *Demodex* est un genre d'acariens de la famille des Demodicidae. Certaines espèces font naturellement partie de la microflore de la peau humaine ou vivent associées aux cheveux, cils, sourcils qui lors de déficit immunitaire pullulent et sont à l'origine de blépharites et de rosacées. Actuellement, seuls les produits mercuriels sont efficaces mais leur toxicité a amené les autorités sanitaires à les retirer du marché.

Concernant les poux, en dehors des produits contenants des insecticides qui sont, comme pour le Demodex, peu à peu délaissés en raison de leur toxicité et de leur inefficacité, il existe des produits qui agissent physiquement en étouffant les poux. Certains de ces produits sont à base de silicone. Ils sont efficaces mais laborieux à appliquer, et ils nécessitent un shampoing et les silicones n'étant pas biodégradables ils ne sont pas bien perçus sur le plan écologique.

D'autres produits anti-poux sont présentés sous forme de microémulsion. Ces produits agissent en étouffant les parasites et non par déshydratation comme les produits à base de sel. Ils sont efficaces, rinçables en une seule étape mais par contre leur prix de revient est élevé.

On sait également que de fortes concentrations en sel permettent d'inhiber la croissance de nombreux microorganismes, dont ceux responsables d'affections cutanées comme par exemple l'acné et les mycoses.

L'acné est la maladie à laquelle le dermatologue est le plus fréquemment confronté qui touche environ 80% des adolescents (dont 15% avec une acné sévère) et près de 25% des adultes, en particulier des femmes. Il s'agit d'une maladie dite auto inflammatoire : elle associe des modifications du fonctionnement normal de la peau et une infection par une bactérie, le *Propionibacterium acneis.*

Les mycoses superficielles cutanées sont dues à des champignons microscopiques. Elles peuvent affecter la surface de la peau, les ongles et le cuir chevelu.

Ce sont les maladies de peau les plus courantes.

Il existe différentes formes de mycoses, selon la partie du corps affectée :
- la mycose des pieds : également surnommée "pied d'athlète", c'est la plus fréquente des mycoses chez l'adulte ; elle s'accompagne souvent d'une atteinte des ongles (onychomycose) ;
- la mycose des mains : elle est similaire mais plus rare et concerne surtout les professions requérant le port de gants ;
- le pityriasis versicolor : il se caractérise par l'apparition, sur le tronc, le cou, le haut des bras, de tâches granuleuses, plus claires que la peau non affectée ; cette infection est courante quel que soit l'âge. Les rechutes sont fréquentes ;
- les mycoses des plis appelées intertrigo : elles siègent dans les plis de l'aine, sous les aisselles, dans les plis des mains.... Le pli est rouge et est le siège de fissures et d'un dépôt jaunâtre.

Il y a 2 causes d'importance majeure d'infections fongiques de la peau et des ongles : les levures et les dermatophytes.

Les infections à levures de la peau et des ongles sont généralement causées par un organisme appelé *Candida albicans.*

Les infections fongiques cutanées peuvent également être causées par le champignon *Pityrosporum orbiculare.* Ces infections, que l'on appelle également *pityriasis versicolor* entraînent la formation de plaques squameuses blanchâtres à brunâtres.

Les produits prescrits pour les mycoses sont à base de molécules antifongiques. Ces produits sont efficaces pour des traitements d'au moins trois semaines. Il s'agit de crèmes grasses et collantes dont la texture est peu agréable.

Selon un autre aspect, on sait également que le sel, en particulier les bains au sel de mer, notamment associés à une exposition aux rayon UV, a un effet bénéfique et permet de diminuer les plaques de psoriasis sur la peau. Il est en effet connu que des cures thermales dans les eaux de la mer morte qui présentent des concentrations salines de l'ordre de trente pourcents ont un effet bénéfique sur les lésions cutanées provoquées par le psoriasis.

L'objectif de la demande est de proposer une solution à base de sel qui résout les problématiques des solutions existantes pour toutes les applications mentionnées.

Pour être utilisé facilement en application topique de façon quotidienne, le sel doit préférentiellement se présenter sous forme de gel. Néanmoins, la gélification du sel n'est pas aisée. En effet, les agents gélifiants sont contrariés par des fortes teneur en sel et les textures obtenues sont en général non satisfaisantes car de viscosité trop faible ce qui rend l'application difficile.

Certains produits contenant du sel sous forme de gel douche notamment ont récemment été fabriqués mais les teneurs en sel sont faibles et non hypertoniques donc non efficace sur les parasites cités plus haut et donc inefficaces pour le traitement des pédiculoses.

On connaît également le brevet US-6607716 qui décrit une composition pédiculicide à base de chlorure de sodium en association avec des tensioactifs, un agent gélifiant et de l'essence d'anis. La concentration en sel revendiquée est faible et pour être efficace le produit doit contenir de l'essence d'anis réputée pédiculicide et agissant comme un insecticide.

Aussi, la présente demande propose une nouvelle composition à base de sel palliant les inconvénients des produits de l'art antérieur, facile à appliquer et efficace pour le traitement de plusieurs désordres et pathologies au niveau cutané.

En particulier, la présente demande vise une composition sous forme de gel, adaptée à une application topique, constituée par :
- entre 1 et 40% de NaCl,
- entre 0,5 et 10% d'hydroxyéthylcellulose ou entre 20 et 50% de lauryl éther sulfate de sodium ou de lauryl sulfate de sodium,
- de l'eau, et
- éventuellement au moins un agent parfumant et/ou au moins un agent kératolytique et/ou au moins une huile essentielle et/ou de la silice colloïdale,
les pourcentages étant donnés en poids par rapport au poids total de la composition.

La composition comprend donc peu de constituants, et est adaptée au traitement de parasitoses cutanées ou dermatites.

Les fortes teneurs en sel contrarient la pousse microbienne et ne nécessitent pas l'ajout de conservateurs ce qui constitue un avantage supplémentaire de la présente invention.

La présente demande concerne également l'utilisation d'une telle composition comme produit à application topique pour la prévention et/ou la lutte contre les poux de tête et/ou contre les lentes, pour la prévention et le traitement de l'acné provoqué par une infection bactérienne, pour la prévention et le traitement des mycoses, des demodex ou du psoriasis. Avantageusement la composition est facile à appliquer sur la peau, les phanères ou les muqueuses, et est très efficace pour déshydrater, asphyxier et éliminer les poux, les *Demodex,* les champignons, les bactéries et pour améliorer l'état des peaux des personnes atteintes de psoriasis.

L'invention est maintenant décrite en détail, notamment en regard d'exemples et de résultats d'essais démontrant son efficacité. La présente invention est définie dans les revendications. Les modes de réalisations qui ne relèvent pas de celles-ci sont uniquement présents à des fins de référence.

L'invention vise donc une composition qui se présente sous forme de gel.

Il s'agit d'une composition adaptée à une application topique.

Elle est constituée par :
- entre 10 et 35% de NaCl,
- entre 1 et 3% d'hydroxyéthylcellulose,
- de l'eau,
   entre 0.1 et 10% de silice colloïdale, et
- éventuellement au moins un agent parfumant et/ou au moins un agent kératolytique et/ou au moins une huile essentielle,
les pourcentages étant donnés en poids par rapport au poids total de la composition.

La composition est constituée par ces seuls éléments (l'agent parfumant et l'huile essentielle n'étant pas obligatoirement présents) et ne comprend pas d'autre composé.

Le NaCl ou chlorure de sodium est un composé chimique ionique de formule NaCl. On l'appelle plus communément sel de table ou de cuisine, ou tout simplement sel dans le langage courant. Il s'agit du principal sel neutre dissous dans l'eau de mer. Il s'obtient dans des salins ou marais salants par évaporation de saumures dans plusieurs bassins communicant avec une réserve remplie par l'eau de mer. C'est pourquoi il s'appelle aussi sel marin. Il peut aussi s'obtenir dans des mines ou salines, par extraction de la roche évaporite saline nommée sel gemme ou halite et plus rarement en le synthétisant, par exemple lors de réaction à hautes températures entre du dichlore (Cl2) et du sodium métallique (Na).

L'eau de mer contient également du NaCl dans des proportions entre 30 à 40 grammes de sels dissous par kilo.

Le chlorure de sodium peut être présent entre 1 et 40% en poids par rapport au poids total de la composition, préférentiellement entre 10 et 35%, encore plus préférentiellement entre 20 et 35%. Selon l'invention le chlorure de sodium est présent entre 10 et 35%.

L'agent gélifiant peut être l'hydroxyéthylcellulose, le lauryl éther sulfate de sodium ou le lauryl sulfate de sodium car seuls ces agents gélifiants, dans des proportions spécifiques, permettent d'obtenir une forme satisfaisante pour une application topique avec des concentrations en sel élevées.

L'hydroxyéthylcellulose est adaptée pour les applications cutanées ou sur les muqueuses, en particulier pour le psoriasis, les demodex, l'acné et les mycoses. Le gel obtenu est transparent, facile à appliquer. Il ne colle pas sur les vêtements et est sans odeur.

L'hydroxyéthylcellulose peut être présent entre 0,5 et 10% en poids par rapport au poids total de la composition. Selon l'invention, l'hydroxyéthylcellulose est présent entre 1 et 3%.

Le lauryl éther sulfate de sodium ou le lauryl sulfate de sodium sont également adaptés jour les applications sur le cuir chevelu ou les phanères, pour les pédiculoses mais également pour les mycoses, le psoriasis.

Dans ce cas, le gel est moussant et peut être utilisé comme shampooing ou gel douche car la présence spécifique de cet agent gélifiant dans les proportions revendiquées, permet également d'obtenir une mousse lorsque le produit est appliqué.

Le lauryl éther sulfate de sodium ou le lauryl sulfate de sodium peut être présent entre 20 et 50% en poids par rapport au poids total de la composition, préférentiellement entre 35 et 45%.

Selon une variante, les préparations peuvent être obtenues directement à partir d'eau de mer à laquelle seront rajoutées le ou les gélifiants préconisé(s) et éventuellement les substances entrant dans l'objet de l'invention comme des parfums, de la silice colloidale ou des essences ou huiles végétales.

La composition selon l'invention comprend également de la silice colloïdale.

Avantageusement la silice colloïdale renforce l'efficacité du produit, en particulier dans le traitement des pédiculoses et du demodex.

La silice peut se présenter sous la forme de silice hydrophile comme l'Aerosil® 200 commercialisé par la société Evonik, ou hydrophobe comme l'Aerosil® R202. En particulier, on préfèrera la famille des oxydes de silicium hydrophiles, par exemple les oxydes de silicium commercialisés sous le nom Aerosil® 90, 130, 150, 200, 300, 388 et particulièrement l'Aerosil® 200.

La silice représente préférentiellement entre 0.1 et 10% en poids par rapport au poids total de la composition.

La composition selon l'invention peut également contenir une ou plusieurs huile(s) ou essence(s) essentielle(s). Il peut s'agir en particulier de nerolidol, d'huile d'arbre à thé, d'essence de myrrhe, de l'essence d'anis, l'huile de céleri.

Ces huiles essentielles permettent de renforcer l'action insecticide et microbicide des formulations et/ou de jouer un rôle répulsif notamment vis-à-vis des insectes comme les poux de tête, de corps et le demodex.

L'huile essentielle est préférentiellement présente entre 0,1 et 5,0% en poids par rapport au poids total de composition.

La composition peut également contenir un ou plusieurs agent(s) kératolytique(s) pour aider à éliminer les cellules mortes de la couche cornée, comme l'urée ou l'acide salicylique par exemple.

Enfin, la composition peut également comprendre un ou plusieurs agent(s) parfumant(s). Il peut s'agir en particulier d'essence de rose de jasmin, d'iris, la violette, le néroli, le mimosa, les narcisses, la lavande, l'ylang-ylang ainsi que des essences reconstituées, par des mélanges de molécules aromatiques synthétiques comme les hespéridés, très présentes dans les eaux de Cologne. Il peut s'agir également des diverses variétés d'essence de citrons et d'oranges, notamment la limette et la bergamote et la vanille, ou encore d'essences et huiles extraites d'écorce (cannelle, santal, cèdre, bouleau, gaïac), ou bien la résine (encens, myrrhe, benjoin, labdanum). On peut citer aussi le romarin, le patchouli, la verveine, le vétiver, la cardamome, la coriandre.

La présence d'agents parfumants est cependant à éviter pour le traitement des dermatites comme le psoriasis, le demodex et l'acné notamment en raison de leur pouvoir allergène.

La composition selon l'invention peut être obtenue par la mise en œuvre d'un procédé comprenant les étapes suivantes, dans le cas où le gel est réalisé à partir d'eau de mer :
- Pesée des matières premières
- Dans la quantité nécessaire d'eau de mer, disperser sous agitation l'agent gélifiant
- Ajouter sous agitation la silice (si nécessaire)
- Ajouter sous agitation le ou les agent(s) kératolytique(s) (si nécessaire)
- Ajouter sous agitation la ou les essence(s) et huile(s) naturelle(s) (si nécessaire)
- Ajouter le ou les parfum(s) (si nécessaire)

La composition selon l'invention peut être obtenue par la mise en œuvre d'un procédé comprenant les étapes suivantes, dans le cas où la formulation est fabriquée à partir de sel cristallin :
- Pesée des matières premières
- Dans la quantité nécessaire d'eau, dissoudre sous agitation le NaCl
- Disperser sous agitation l'agent gélifiant
- Ajouter sous agitation la silice (si nécessaire)
- Ajouter sous agitation le ou les agent(s) kératolytique(s) (si nécessaire)
- Ajouter sous agitation les essences et huiles naturelles (si nécessaire)
- Ajouter les parfums (si nécessaire)

La composition obtenue se présente sous forme de gel. Elle est administrable sous forme de pot, de tube ou de spray.

La composition peut être utilisée en application topique sur la peau, les phanères, les muqueuses ou le cuir chevelu, dans la prévention et le traitement des parasitoses et des affections dermatologiques en général.

En particulier la composition selon l'invention est utilisée pour la prévention et le traitement des pédiculoses, notamment des pédiculoses de la tête. Appliquée sur le cuir chevelu infesté, elle est très efficace pour éliminer à la fois les poux et les lentes. Elle agit principalement en déshydratant les poux et les lentes. La présence de sel permet de pomper l'eau par choc osmotique et le poux ou la lente meurent asphyxiés.

Préférentiellement la composition est appliquée sur le cuir chevelu pendant une durée comprise entre 5 minutes et 24 heures, notamment entre 15 minutes et huit heures. Le cuir chevelu et la chevelure sont ensuite rincés à l'eau claire pour éliminer lentes et poux morts.

La composition peut également être utilisée en application topique sur la peau, pour la prévention et le traitement de l'acné provoqué par une infection bactérienne mais également pour les mycoses.

Il a été démontré au cours des essais exposés dans la présente demande que les préparations issues de l'invention présentent intrinsèquement une absence de contamination microbienne. Ceci s'explique en particulier par le caractère hyper osmotique des préparations.

Le mode d'application préconisé est d'appliquer le gel sur le cuir chevelu ou la peau, de masser doucement et de le laisser en place pendant l'espace d'une nuit.

La composition peut également être utilisée en application topique sur la peau, pour la prévention et le traitement du demodex.

A l'instar du phénomène observé pour les poux, l'application de solutions hyperosmotique provoque la déshydratation des parasites.

La forme gélifiée permet une application précise sur les pourtours de l'œil, les paupières.

La composition est aussi particulièrement efficace en application topique sur la peau ou le cuir chevelu, pour la prévention et le traitement du psoriasis.

Le gel facile d'application permet de recréer la concentration en sel observée sur les côtes de la mer morte. On observe après application du gel sur des lésions du psoriasis une hydratation particulière de la peau. On peut soit appliquer le gel seul soit appliquer ensuite une crème grasse pour compléter le traitement par exemple à base d'acide salicylique, d'urée, de vitamine D ou de corticoïdes. Huit jours après l'application on constate une diminution de l'intensité des lésions.

Les caractéristiques et avantages de l'invention peuvent être illustrés par des exemples non limitatifs et des essais. Les exemples 1-5 sont présentés à titre illustratif.

### EXEMPLE 1 : FORMULATION ADAPTEE POUR ANTI POUX ET DEMODEX

| **Composants** | **% d'incorporation P/P** | **Fonction** |
|---|---|---|
| Eau | 49 | Solvant |
| Texapon N 70 | 40 | Agent gélifiant et tensioactif |
| Sodium Chloride | 10 | Agent osmotique |
| Nerolidol | 1 | Parfum |

Le procédé de fabrication de cette composition consiste à dissoudre le sel dans la phase aqueuse sous agitation lente et d'introduire les autres composants de façon à obtenir un gel homogène.

### EXEMPLE 2 : FORMULATION ADAPTEE POUR ANTI POUX ET DEMODEX

| **Composants** | **% d'incorporation P/P** | **fonction** |
|---|---|---|
| Eau | 47 | Solvant |
| Texapon N 70 | 40 | Agent gélifiant et tensioactif |
| Sodium Chloride | 10 | Agent osmotique |
| Nerolidol | 1 | Parfum |
| Silice colloïdale (Aerosil 200) | 2 | Agent de texture |

Le procédé de fabrication de cette composition consiste à dissoudre le sel dans la phase aqueuse sous agitation lente et d'introduire les autres composants de façon à obtenir un gel homogène.

### EXEMPLE 3 : FORMULATION ADAPTEE POUR PSORIASIS, MYCOSE OU ACNE

| **Composants** | **% d'incorporation P/P** | **fonction** |
|---|---|---|
| Eau | 67 | Solvant |
| Hydroxyéthylcellulose | 3 | Agent gélifiant |
| Sodium Chloride | 30 | Agent osmotique |

Le procédé de fabrication consiste à dissoudre le sel dans la phase aqueuse sous agitation lente et d'introduire ensuite l'hydroxyethylcellulose et les autres composants de façon à obtenir un gel homogène.

### EXEMPLE 4 : FORMULATION ADAPTEE POUR PSORIASIS, MYCOSE ou ACNE

| **Composants** | **% d'incorporation P/P** | **fonction** |
|---|---|---|
| Eau | 57 | Solvant |
| Hydroxyéthylcellulose | 3 | Agent gélifiant |
| Sodium Chloride | 30 | Agent osmotique |
| Urée | 10 | Agent Kératolytique |

Le procédé de fabrication consiste à dissoudre le sel dans la phase aqueuse sous agitation lente et d'introduire ensuite l'hydroxyethylcellulose et les autres composants de façon à obtenir un gel homogène.

### EXEMPLE 5 : GEL MOUSSANT ADAPTE POUR PSORIASIS, MYCOSE OU ACNE

| **Composants** | **% d'incorporation P/P** | **fonction** |
|---|---|---|
| Eau | 65 | Solvant |
| Hydroxyethylcellulose | 3 | Agent gélifiant |
| Sodium Chloride | 30 | Agent osmotique |
| Texapon N70 | 2 | Agent gélifiant et moussant |

Le procédé de fabrication de cette composition consiste à dissoudre le sel dans la phase aqueuse sous agitation lente et d'introduire ensuite l'hydroxyethylcellulose et les autres composants de façon à obtenir un gel homogène.

Des tests comparatifs concernant le choix des gélifiants d'une part, et des tests d'efficacité d'autre part, ont été menés sur ces compositions.

### EVALUATION DE L'IMPORTANCE DES AGENTS GELIFIANTS SELON L'INVENTION

### Importance de la présence d'au moins un agent gélifiant

Un essai comparatif sur l'effet pédiculicide et lenticide d'une composition selon l'invention sous forme de gel comparée à une forme liquide a été réalisé.

Les formules testées sont les suivantes :
Forme liquide (Composition 1) :

| **Composants** | **% d'incorporation P/P** |
|---|---|
| Eau | 75,96 |
| Hydroxyéthylcellulose | 0 |
| Chlorure de sodium | 10,36 |
| Diglycérine | 4,73 |
| laureth sulfate de Sodium | 3,37 |
| Glycereth-7cocoate | 2,00 |
| Cocamidopropylbetaine | 1,80 |
| Tea tree oil | 0,50 |
| Silice | 0,50 |
| chlorure de'amidon hydroxypropyltrimonium | 0,36 |
| Glycérine | 0,25 |
| Lactate de sodium | 0,05 |
| Urée | 0,04 |
| Acide lactique | 0,04 |
| Hydroxyde de potassium | 0,03 |
| Acide benzoïque | 0,01 |

Forme gélifiée (Composition 2) - invention :

| **Composants** | **% d'incorporation P/P** |
|---|---|
| Eau | 74,76 |
| Hydroxyéthylcellulose | 1,20 |
| Chlorure de sodium | 10,36 |
| Diglycérine | 4,73 |
| laureth sulfate de Sodium | 3,37 |
| Glycereth-7cocoate | 2,00 |
| Cocamidopropylbetaine | 1,80 |
| Tea tree oil | 0,50 |
| Silice | 0,50 |
| chlorure de'amidon hydroxypropyltrimonium | 0,36 |
| Glycérine | 0,25 |
| Lactate de sodium | 0,05 |
| Urée | 0,04 |
| Acide lactique | 0,04 |
| Hydroxyde de potassium | 0,03 |
| Acide benzoïque | 0,01 |

L'effet pédiculicide et lenticide des formulations a été testé en laboratoire sur des poux humains.

Des poux (*Pediculus humanus*) ont été élevés en laboratoire selon la méthode de Cole (1966). Pour chaque test, 50 poux (10 males et 10 femelles et 30 nymphes) sont disposés sur un filtre de papier blanc en forme de disque de 7 cm de diamètre, et exposés à 1g de la formulation test. Les poux ont été laissés en contact avec la substance pendant 15 minutes. Ils ont ensuite été enlevés du papier filtre, lavés avec un shampoing normal (1 :20) pendant 1 minute, puis avec de l'eau pendant 1 minute. Après traitement les poux ont été transférés sur un nouveau filtre de papier blanc en forme de disque et incubés. Le taux de morbidité a été déterminé après 24 heures.

Par ailleurs des poux ont été placés sur des cheveux humains et 50 œufs de 2 à 6 jours ont été récupérés puis traités comme les poux. Le nombre de poux éclos et non éclos est compté au bout de 10 jours.

Un contrôle négatif est réalisé en traitant les poux avec de l'alcool éthylique 40%.

Chaque temps d'exposition a été réalisé en triplicata.

Les résultats sont présentés dans le tableau ci-dessous :

| Formulation | Taux moyen de morbidité de poux en pourcentage | Taux moyen de morbidité de lentes en pourcentage |
|---|---|---|
| Composition 1 | 65 | 50 |
| Composition 2 (invention) | 100 | 100 |
| Contrôle | 8 | 12 |

On constate que la forme gélifiée possède une bien meilleure efficacité qu'une composition hypertonique sans principe actif sous forme liquide.

### Importance du choix des agents gélifiants

Différents gélifiants ont été mis en œuvre pour tenter de gélifier des solutions contenant des proportions de NaCl comprises entre 10 et 30%.

Le protocole opératoire consiste à dissoudre le NaCl dans l'eau sous agitation, puis à ajouter le gélifiant aux concentrations usuelles d'utilisation.

La viscosité est mesurée à l'aide d'un appareil à mobile tournant conforme à ceux décrits dans la Pharmacopée. Elle est exprimée en centipoise.

La mesure est réalisée à 25°C.

Le tableau ci-après résume les résultats obtenus pour des solutions salines à 30% :

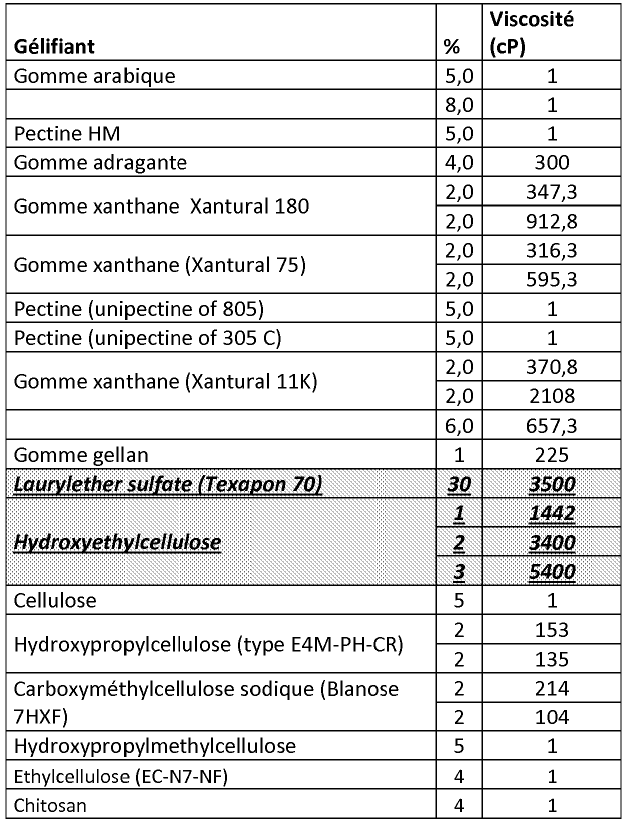

A sa lecture on constate que seul l'hydroxyéthylcellulose et le Texapon 70 sont capables de gélifier des solutions de NaCl à des concentrations aussi élevées que 30%.

Les gels obtenus sont transparents cristallins.

On constate également aucune pousse bactérienne sur ces gels contrairement aux formulations mettant en œuvre les autres gélifiants.

### EVALUATION DE L'EFFICACITE PEDICULICIDE ET LENTICIDE DE L'EXEMPLE 1

### Poux

Cette étude réalisée ex-vivo, consiste à mettre en contact (temps de contact illimité) des poux avec le produit selon l'invention de l'exemple 1 d'une part et avec un témoin « eau distillée » d'autre part, et à observer les poux (morts, affaiblis = « knock-down » et vivants) à 5min, 15 min, 30 min, 35 min, 1h, 3h et 24h.

Les résultats sont présentés dans le tableau ci-après :

| | | **POUX** | | | | | |
|---|---|---|---|---|---|---|---|
| | | ***Morts*** | | ***Knock-Down*** | | ***Vivants*** | |
| *Temps écoulé pendant **contact** illimité* | **Solut° testée** / nbre poux testé | ***Nombre*** | ***%*** | ***Nombre*** | ***%*** | ***Nombre*** | **%** |
| ***5 min*** | ***Témoin H₂O* /*43px*** | ***0*** | ***0*** | ***43⁺*** | ***100*** | ***0*** | ***0*** |
| | ***Invention Ex1*/ *52px*** | ***0*** | ***0*** | ***52³⁺*** | ***100*** | ***0*** | ***0*** |
| ***15 min*** | ***Témoin H₂O* /*43px*** | ***0*** | ***0*** | ***0*** | ***0*** | **43** | ***100*** |
| | ***Invention Ex1*/ *52px*** | ***0*** | ***0*** | ***52⁴⁺*** | ***100*** | ***0*** | ***0*** |
| ***30 min*** | ***Témoin H₂O* /*43px*** | ***0*** | ***0*** | ***0*** | ***0*** | **43** | ***100*** |
| | ***Invention Ex1*/ *52px*** | **52** | ***100*** | ***0*** | ***0*** | ***0*** | ***0*** |
| ***45 min*** | ***Témoin H₂O* /*43px*** | ***0*** | ***0*** | ***0*** | ***0*** | **43** | ***100*** |
| | ***Invention Ex1*/ *52px*** | ***52*** | ***100*** | ***0*** | ***0*** | ***0*** | ***0*** |
| ***1 h*** | ***Témoin H₂O* /*43px*** | ***1*** | ***2,30*** | ***0*** | ***0*** | ***42*** | ***97,70*** |
| | ***Invention Ex1*/ *52px*** | ***52*** | ***100*** | ***0*** | ***0*** | ***0*** | ***0*** |
| ***3 h*** | ***Témoin H₂O* /*43px*** | **1** | ***2,30*** | ***0*** | ***0*** | **42** | ***97,70*** |
| | ***Invention Ex1*/ *52px*** | ***52*** | ***100*** | ***0*** | ***0*** | ***0*** | ***0*** |
| ***24 h*** | ***Témoin H₂O* / *43px*** | ***31*** | ***72,10*** | ***1³⁺*** | ***2,30*** | ***11*** | ***25,60*** |
| | ***Invention Ex1*/ *52px*** | ***52*** | ***100*** | ***0*** | ***0*** | ***0*** | ***0*** |

### Lentes

Cette étude réalisée ex-vivo, consiste à mettre en contact 15 minutes des lentes pendant l'incubation de 12 jours des lentes à 34°C et 48%RH, avec le produit selon l'invention de l'exemple 1 d'une part et avec un témoin « eau distillée » d'autre part, et à observer les lentes (écloses, mortes en cours d'éclosion et non écloses).

Les résultats sont présentés dans le tableau ci-après :

| | | **Produits testés / lentes totales** | | | |
|---|---|---|---|---|---|
| | | **Témoin eau /107** | | ***Invention Ex1* /157** | |
| | | **Nbre** | **%** | **Nbre** | **%** |
| **Lentes** | **écloses** | **72** | **67,30** | **0** | **0** |
| | **mortes en cours d'éclosion** | **2** | **1,85** | **2** | **1,27** |
| | **NON écloses** | **33** | **30,85** | **155** | **98,70** |

On constate sur des poux de tête une mortalité totale des parasites après un temps de contact de 30 minutes ce qui est remarquablement rapide.

Après un temps de contact de seulement quinze minutes 100% des lentes sont éliminées.

## Revendications

1. Composition sous forme de gel, adaptée à une application topique, constituée par :
- entre 10 et 35% de NaCl,
- entre 1 et 3% d'hydroxyéthylcellulose,
- de l'eau,
- entre 0,1 et 10% de silice colloïdale, et
- éventuellement au moins un agent parfumant et/ou au moins un agent kératolytique et/ou au moins une huile essentielle,
les pourcentages étant donnés en poids par rapport au poids total de la composition.

2. Composition selon la précédente revendication, **caractérisée en ce qu'**il s'agit d'un gel administrable sous forme de tube ou de spray.

3. Composition selon l'une des précédentes revendications pour son utilisation dans la prévention et le traitement des pédiculoses.

## Patentansprüche

1. Zusammensetzung in Gelform zur topischen Anwendung, bestehend aus:
- 10 bis 35 % NaCl,
- 1 bis 3 % Hydroxyethylcellulose,
- Wasser,
- 0,1 bis 10 % kolloidaler Kieselsäure und
- gegebenenfalls mindestens einem Duftstoff und/oder mindestens einem keratolytischen Mittel und/oder mindestens einem ätherischen Öl, wobei die Prozentzahlen in Gewichtsprozent, bezogen auf das Gesamtgewicht der Zusammensetzung, angegeben sind.

2. Zusammensetzung nach dem vorhergehenden Anspruch, **dadurch gekennzeichnet, dass** es sich um ein Gel handelt, das in Tuben- oder Sprayform verabreicht werden kann.

3. Zusammensetzung nach einem der vorhergehenden Ansprüche zur Verwendung bei der Prävention und Behandlung von Pedikulose.

## Claims

1. Composition in gel form, suitable for topical application, consisting of:
- between 10 and 35% NaCl,
- between 1 and 3% hydroxyethyl cellulose,
- water,
- between 0.1 and 10% colloidal silica, and
- optionally at least one perfuming agent and/or at least one keratolytic agent and/or at least one essential oil,
the percentages being given by weight relative to the total weight of the composition.

2. Composition according to the preceding claim, **characterized in that** said composition is a gel which can be administered in the form of a tube or a spray.

3. Composition according to either of the preceding claims for use in the prevention and treatment of pediculosis.
